# EUROPÄISCHE PATENTSCHRIFT

(11) **EP 1 402 045 B1**
(45) Veröffentlichungstag und Bekanntmachung des Hinweises auf die Patenterteilung: **07.07.2010**
(21) Anmeldenummer: 02716743.6
(22) Anmeldetag: 08.02.2002
(51) Int. Cl.: C12P 7/64, C12R 1/90

(54) **VERFAHREN ZUR HERSTELLUNG VON GAMMA-LINOLENSÄURE AUS EINER CILIATEN-KULTUR DURCH ZUSATZ GEEIGNETER VORLÄUFERMOLEKULE ZU DEM KULTURMEDIUM**
METHOD FOR PRODUCING G(G)-LINOLENIC ACIDS FROM A CILIATE CULTURE BY ADDING SUITABLE PRECURSOR MOLECULES TO SAID CULTURE MEDIUM
PROCEDE POUR PRODUIRE DES ACIDES G(G)-LINOLENIQUES A PARTIR D'UNE CULTURE DE CILIES PAR ADDITION DANS CE MILIEU DE CULTURE DE MOLECULES PRECURSEURS ADAPTEES

(30) Priorität: 12.02.2001 DE 10106660
(43) Veröffentlichungstag der Anmeldung: 31.03.2004
(73) Patentinhaber: Nutrinova Nutrition Specialties & Food Ingredients GmbH, 65926 Frankfurt am Main (DE)
(72) Erfinder: VAN PUTTEN, Anton, NL-2497 AC Den Haag (NL); KÖNIG, Tanja, 13359 Berlin (DE); FABRITIUS, Dirk, 60529 Frankfurt (DE); KIY, Thomas, 65929 Frankfurt (DE); LESKE, Alexander, 60529 Frankfurt (DE); PLOTE, Joern, 65719 Hofheim/Ts. (DE)
(74) Vertreter: Luderschmidt, Schüler & Partner
(86) Internationale Anmeldenummer: PCT/EP2002/001302
(87) Internationale Veröffentlichungsnummer: WO 2002/064807

(56) Entgegenhaltungen:
- EP-A- 0 252 716
- WO-A-86/03518
- DATABASE CA [Online] CHEMICAL ABSTRACTS SERVICE, COLUMBUS, OHIO, US; KOROLY, MARY J. ET AL: "Unsaturated fatty acid biosynthesis in Tetrahymena. Evidence for two pathways" retrieved from STN Database accession no. 86:52528 XP002218552 & J. BIOL. CHEM. (1976), 251(23), 7588-92 ,
- DATABASE BIOSIS [Online] BIOSCIENCES INFORMATION SERVICE, PHILADELPHIA, PA, US; 1980 NOZAWA Y ET AL: "MODIFICATIONS OF MEMBRANE LIPID COMPOSITION FOLLOWING THE NUTRITIONAL SHIFT-UP OF STARVED CELLS A COMPARISON WITH MEMBRANE BIOGENESIS IN TETRAHYMENA-PYRIFORMIS" Database accession no. PREV198171060198 XP002218553 & BIOCHIMICA ET BIOPHYSICA ACTA, Bd. 603, Nr. 2, 1980, Seiten 347-365, ISSN: 0006-3002
- HERTER CH. A. ET AL: 'Lipid Metabolism of Ciliated Protozoa' THE JOURNAL OF BIOLOGICAL CHEMISTRY Bd. 238, Nr. 4-6, 05 Mai 1963, BALTIMORE, Seiten 1189 - 2254
- HILL D.L. THE BIOCHEMISTY AND PHYSIOLOGY OF TETRAHYMENA 1972, NEW YORK, LONDON, Seiten 46 - 73

## Beschreibung

Die Erfindung betrifft ein Verfahren zur fermentativen Herstellung von omega-6-Fettsäuren (PUFA, polyunsaturated fatty acids), insbesondere γ-Linolensäure (GLA) in Ciliaten. Die Lipide/Fettsäuren können in der menschlichen/tierischen Ernährung, sowie im Pharmabereich zum Einsatz kommen.

Die Ciliaten, aus denen die Lipide/Fettsäuren gewonnen werden, sind vorzugsweise *Tetrahymena spec.* und besonders bevorzugt *Tetrahymena thermophila.*

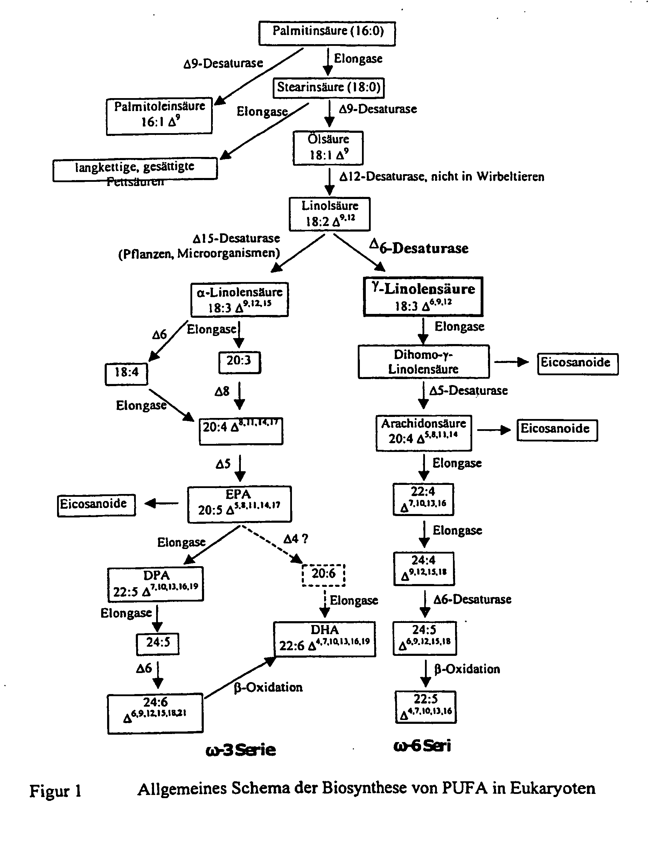

In Figur 1 ist ein allgemeines Schema der Biosynthese von PUFAs unter Berücksichtigung der beteiligten Enzyme in Eukaryoten (modifiziert nach Gill & Valivety, Trends Biotechnol. 1997, 15:401-409) dargestellt. Die Umwandlung von Stearinsäure (18:0) zu Ölsäure (18:1 Δ9) wird durch eine delta 9-Desaturase katalysiert. Ölsäure wird durch eine delta 12-Desaturase in Linolsäure (18:2 Δ 9,12; kurz LA) umgewandelt, die wiederum durch eine delta 6-Desaturase in γ-Linolensäure (18:3, Δ6,9,12; kurz: GLA), bzw. durch eine delta 15-Desaturase in α-Linolensäure (18:3 Δ9,12,15; kurz: ALA) umgewandelt wird. Die Verlängerung der Fettsäuren wird durch Elongasen katalysiert, wodurch z. B. aus γ-Linolensäure Dihomo-γ-Linolensäure (20:3 Δ8,11,15; kurz: DGLA) gebildet wird, die wiederum durch eine delta 5-Desaturase zu Arachidonsäure (20:4 Δ5,8,11,15; kurz: ARA) umgewandelt wird, einem direkten Vorläufermoleküle physiologisch wirksamer Eicosanoide, wie z. B. Prostaglandine, Prostacycline, Thromboxane, Leukotriene. Bei der Bildung der PUFAs, die sich von GLA ableiten (nachfolgend delta 6-ungesättigte Fettsäuren genannt), hat sich gezeigt, daß die Umwandlung von LA zu GLA durch die delta 6-Desaturase der limitierende Schritt ist (Huang YS & Mills DE, γ-linolenic acid: Metabolism and its role in nutrition and medicin. AOCS Press, Champaign, Illinois, 1996).

Da Vertebraten keine Doppelbindungen hinter Position 9 in Fettsäuren einfügen können, sind ungesättigte Fettsäuren wie LA und ALA essentielle Nährstoffe, die von Vertebraten nicht synthetisiert werden können, und in der Ernährung hauptsächlich aus pflanzlichen Quellen stammen. LA kann von Säugern durch eine delta 6-Desaturase in GLA umgewandelt werden - eine ARA-Vorstufe - das ein essentielles Vorläufermolekül der meisten Prostaglandine ist. Die Bildung von Stearidonsäure (18:4 Δ6,9,12,15) - einem Vorläufermolekül der EPA - aus ALA wird ebenfalls mittels delta 6-Desaturase katalysiert. Die delta 6-Desaturase ist damit der erste essentielle Schritt in der Biosynthese der Eicosanoide.

Es hat sich gezeigt, daß die Aktivität der delta 6-Desaturase bei Säugern durch Faktoren wie z. B. Alkoholkonsum, Streß, Mangelernährung und Alterungsprozesse beeinträchtigt wird (Huang & Mills, 1996; Horrobin (1990) Rev. Contemp. Pharmacother. 1: 1-45; Bolton-Smith C et al. (1997) Eur. J. Clin. Nutr. 51:619-624; Leventhal L J et al. (1993) Ann. Intern. Med. 119:867-873). Dies führt zu einer Unterversorgung mit GLA und damit letztlich zu einem Mangel an den GLA abgeleiteten Moleküle wie ARA und der daraus gebildeten physiologisch wichtigen Eicosanoide, (Brenner RR (1976) Adv. Exp. Med. Biol. 83:85-101; Nakahara T et al. (1993) J. Jpn. Oil Chem. Soc. 42:242-253; Chapkin, RS (1998) Reappraisal of the essential fatty acids. In: Fatty acids in food and their health implications, 2nd ed. (Chow CK, ed) Marcel Dekker, New York, NY). Die Zuführung von GLA kann sowohl einen reduzierten endogenen Spiegel von delta-6 ungesättigten Fettsäuren ausgleichen, als auch einen erhöhten Bedarf an diesen Fettsäuren decken (Horrobin (1990)). Für die Biosynthese von aus GLA abgeleiteten Molekülen ist deshalb die Aufnahme von GLA durch die Nahrung vorteilhaft (Fan, YY & Chapkin, RS (1998) J. Nutr, 128:1411-1414).

Der Befund, daß GLA vielfältige positive Einflüsse auf den menschlichen Körper ausübt, wurde inzwischen durch eine Vielzahl wissenschaftlicher Studien untermauert. So wurde die positive Wirkung der GLA z. B. auf atopische Ekzeme, Rheumatische Arthritis, Atheriosklerose, diabetische Neuropathie, Migräne, Schizophrenie und Krebs durch klinische Studien nachgewiesen. In diesen Studien wurde sowohl eine statistisch als auch eine klinisch signifikante Verbesserung des Krankheitsbildes erzielt.

Aufgrund dieser positiven Eigenschaften besteht ein breites Anwendungsspektrum für GLA in der Pharma-, Kosmetik-, Tierfutter- und Lebensmittelindustrie

Die meisten PUFA aus Menschen und Tieren stammen entweder direkt aus der Ernährung oder entstehen durch die Umsetzung der durch die Ernährung zugeführten essentiellen Fettsäuren durch Desaturasen und Elongasen.

Nur wenige der kommerziell genutzten Ölsaaten produzieren GLA. GLA kommt nur im Samen verschiedener Pflanzen wie der Nachtkerze *(Oenothera biennis,* ca. 10% GLA, bezogen auf den Gesamtfettsäuregehalt), Borretsch *(Borago officinalis,* ca. 23%) und der Schwarzen Johannisbeere *(Ribes nigrum,* ca. 18%) vor. Daneben sind auch verschiedene Mikroorganismen als Quellen für GLA bekannt, wie z. B. die Pilze *Mucor* und *Mortierella* (bis zu ca. 25%), die Blaualge *Spirulina* (ca. 12-18%) und andere. Als besonders GLA-reiche Quelle wurden Ciliaten wie z. B. *Tetrahymena* (bis zu 47%; Hill, DL (1972) The biochemistry and physiology of Tetrahymena. Chapter 3, 46-73. Academic press, New York, London; Erwin, J & Bloch, K (1963) J. Biol. Chem. 238:1618-1624) beschrieben.

Die fermentative Gewinnung von GLA aus verschiedenen Pilze wie z.B. *Mortierella* und *Mucor* wurde in der Literatur bereits beschrieben (Nakajima et al, Essent. Fatty Aicosanoids, Invited Pap. Int. Congr., 3rd, 1992, 57-64; Nakahara et al, Industrial appl. of single cell oils, AOCS Press, (1992), 61-97). Eine ausreichende Sauerstoffversorgung, die essentiell für die GLA-Produktion ist, läßt sich aufgrund der bei filamentös wachsenden Pilzen üblichen hohen Viskosität des Kulturmediums im industriellen Maßstab nur unter hohem Aufwand verwirklichen, und dieses Problem harrt noch immer seiner Überwindung (Hiruta et al., j. Ferm. Biotech., (1997), 83, 79-86; Chmiel, Bioprozeßtechnik 2, (1991), 287-302; Crueger & crueger, Biotechnologie - Lehrbuch der angewandten Mikrobiologie, (1989), 224-266).

Die kommerzielle Gewinnung von GLA aus diesen natürlichen Quellen ist also mit schwerwiegenden Nachteilen und Problemstellungen behaftet, die eine Nutzung in industriellem Maßstab bisher verhindert haben. Sowohl die Qualität als auch die Quantität der aus diesen Organismen gewonnen Öle schwanken, und die Öle weisen eine sehr heterogene Zusammensetzung auf, was aufwendige und teure Reinigungsschritte nötig macht, um die GLA anzureichern. Der Anbau GLA-haltiger Pflanzen ist darüberhinaus nicht sehr wirtschaftlich (Hansen CE et al. (1991) J. Sci. Food Agric. 54:309-312). Für die Gewinnung von GLA-haltigem Öl hat sich gezeigt, daß die Raum/Zeit-Ausbeute bei einigen GLA-produzierenden Mikroorganismen im Vergleich zu höheren Pflanzen deutlich besser ist.

Aus diesem Grunde bietet die fermentative Herstellung von GLA durch Mikroorganismen eine an sich vielversprechende Alternative zu anderen GLA-Quellen, die aber bisher nicht in zufriedenstellendem Maße verwirklicht werden konnte.

Das Fettsäurespektrum vieler Mikroorganismen ist weniger heterogen als bei höheren Organismen, was bei der Reinigung Vorteile bietet. Darüber hinaus ist die fermentative Herstellung nicht von externen Faktoren wie Wetter, Nahrungsangebot etc. abhängig. Außerdem sind auf diese Weise hergestellte PUFA weitgehend frei von Kontaminationen, die z.B. auf Umweltverschmutzung zurückzuführen sind. Ein weiterer Vorteil ist die im Gegensatz zu GLA aus z.B. pflanzlichen Quellen gesicherte Verfügbarkeit der durch fermentative Prozesse gewonnenen GLA.

Aufgrund des immensen Interesses, PUFA bzw. GLA industriell zur Verfügung zu stellen, gibt es im Stand der Technik eine Fülle von Versuches, die fermentative Produktion dieser Fettsäuren zu optimieren. Beispiele hierfür sind zu finden in Ratledge C (1993) Trends Biochem. 11:278-284; Ratledge C (1989) Biochem. Soc. Trans. 17:1139-1141, Gosselin Y et al. (1989) Biotechnol. Lett. 11:423-426 sowie der Internationalen Patentanmeldung WO 86/03518). Für eine fermentative GLA-Produktion durch Mikroorganismen im industriellen Maßstab ist aber eine Steigerung des GLA-Gehaltes notwendig, da die Fermentation PUFA-produzierender Mikroorganismen aufwendig und teuer und demzufolge bei den zur Zeit erreichbaren GLA-Gehalten zur Zeit unwirtschaftlich ist (supra Ratledge 1993). Außerdem ist aufgrund des geringen GLA-Gehalts bezogen auf die Gesamtlipide eine Reinigung vonnöten, die nicht quantitativ durchgeführt werden kann, so daß die Darstellung von hochreiner GLA aus fermentativer Herstellung zur Zeit unter praktischen Gesichtspunkten in nennenswerten Ausbeuten nicht möglich ist.

Für den Mikroorganismus *Mortierella* konnte gezeigt werden, dass die Zugabe von Kohlenwasserstoffen in das Kulturmedium zu einer Konzentrationserhöhung der von *Mortierella* produzierten Dihomo-γ-Linolensäure führt (EP 0 252 716 A2).

Ferner wurden bereits zur Untersuchung der Fettsäure-Biosynthese in *Tetrahymena Tetrahymena pyriformis* in Gegenwart von Palmitoleinsäure bzw. Stearinsäure kultiviert und die von den Mikroorganismen produzierten Lipide isoliert (Koroly et al., J. Biol. Chem., (1976) 251, 7588-7592. Die dabei erhaltenen Ergebnisse trugen zum Verständnis der Biosynthesewege ungesättigter Fettsäuren in *Tetrahymena* bei.

Aufgrund seines natürlicherweise bereits relativ hohen GLA-Gehalts könnte besonders *Tetrahymena* für eine fermentative Gewinnung von GLA geeignet sein. *Tetrahymena* kann im Fermenter kultiviert werden und es können hohe Zelldichten erreicht werden (Kiy, T. & Tiedtke (1992) Appl. Microbiol. Biotechnol. 37, 576-579; Kiy, T. & Tiedtke, A. (1992) Appl. Microbiol. Biotechnol. 38, 141-146).

PUFA (dazu gehört GLA) liegen.in *Tetrahymena* überwiegend gebunden in den Phospholipiden (PL) der Zellmembranen vor. Die Zellen beinhalten 3,5 bis 4 mal mehr Phospholipid als Neutrallipid (Speicherlipide) (Lees et al, Biochemistry, (1966) 5(5), 1475-1481, Jonah et al., (1971) Biochimica et biophysica acta, 231, 80-92). Die Fläche der Zellmebranen ist jedoch begrenzt und kann in Kultur nur über die Steigerung der Zellzahl erhöht werden. Dadurch wird einer Steigerung des PUFA-Gehaltes eine natürliche Grenze gesetzt, da die Zellzahl in Kultur nicht beliebig gesteigert werden kann.

Die Menge der Neutrallipide (Speicherlipide) pro Zelle kann hingegen gesteigert und in Form von Lipidtröpfchen akkumuliert werden. Dies ist für die gezielte Fütterung von Acetat beschrieben worden (Okuyama et al., J. Biol. Chem., (1978) 253 (10), 3588-3594; Borowitz et al., Biochim. et biophys. acta, (1976) 424, 114-124).

Der Anteil an GLA in den Neutrallipiden (NL) ist allerdings sehr gering (6,7 % (w/w) im NL zu 25,6 % (w/w) im Gesarntlipid (Jonah et al., Biochimica et biophysica acta, (1971) 231, 80-92) bzw. 2,1 % (w/w) in den NL zu-29,5 % (w/w) in den Phospholipiden (PL) und 19 % (w/w) im Gesamtlipid (Erwin et al. J. Biol. Chem., (1963) 238 (5), 1618-1624)). Die Zusammensetzung der Fettsäuren ändert sich durch die Zufütterung mit Acetat nicht (Holz et al., (1973), Biol Tetrahymena, 99-122).

Für *Tetrahymena pyriformis* wird ein Anstieg der Zellzahl bei der Zufütterung von Acetat beschrieben (von 1,2x10⁵ auf 1,6x10⁵ Zellen pro mL bei der Zugabe von 0,1% Acetat zu einem basalen Medium, das keine Glucose beinhaltet. Bei einem basalen Medium, das Glucose beinhaltet, ist der Anstieg nicht signifikant (1,85x10⁵ ohne bzw. 1,9x10⁵ Zellen pro mL mit Acetat) (Mavrides, (1973), Can. J. Biochem, 323-331, Medium composition: Dewey et al, (1950) Arch. Biochem. Biophys, 29, 281). Die in diesen Veröffentlichungen angegebenen Zellzahlen liegen allerdings weit unter den Zellzahlen, die mit den erfindungsgemäß verwendbaren Medien (maximal 2x10⁵ gegenüber 1x10⁶ -3x10⁷ in dem in der Erfindung benutzten Medium) erreicht werden können.

Der vorliegenden Erfindung liegt daher die Aufgabe zugrunde, ein Verfahren zur Herstellung von PUFA, bevorzugt GLA, zur Verfügung zu stellen, mit dem deutlich erhöhte GLA-Ausbeuten in industriellem Maßstab erreicht werden können. Dieses Verfahren soll technisch unkompliziert, einfach in der Handhabung, sicher und preiswert sein.

Eine weitere Aufgabe der vorliegenden Erfindung war es, den GLA-Gehalt im Neutrallipid zu steigern, um die GLA in einer reineren Form zu erhalten.

Gelöst werden diese sowie weitere, nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen des erfindungsgemäßen Verfahrens werden in den auf Anspruch 1 rückbezogenen Unteransprüchen unter Schutz gestellt.

Dadurch, daß man Ciliaten unter an sich bekannten Bedingungen im Fermenter kultiviert und GLA-Vorläufermoleküle und Acetat dem Kulturmedium zusetzt, kann nämlich in nicht vorhersehbarer Weise eine erhöhte Ausbeute an GLA erreicht werden.

In einer bevorzugten Ausführungsform der vorliegenden Erfindung handelt es sich bei dem Ciliaten um *Tetrahymena spec.,*

In einer weiteren bevorzugten Ausführungsform der vorliegenden Erfindung handelt es sich bei dem Ciliaten *Tetrahymena rostrata (z. B. ATCC 30770)*

In noch einer weiteren bevorzugten Ausführungsform der vorliegenden Erfindung handelt es sich bei dem Ciliaten um *Tetrahymena pyriformis (z. B. A TCC 30039, 30005, 30202).*

In noch einer weiteren bevorzugten Ausführungsform der vorliegenden Erfindung handelt es sich bei dem Ciliaten um *Tetrahymena thermophila B1868 VII (z. B. ATCC 30384)*

Bevorzugt ist es, wenn das Acetat erst 20-39 Stunden nach Kulturbeginn zugegeben wird, besonders bevorzugt, wenn es erst nach 25-35 Stunden zugegeben wird.

In einer weiteren bevorzugten Ausführungsform der vorliegenden Erfindung wird das Acetat 2-stufig zuzugeben wird.

Daher ist es eine besonders bevorzugte Ausführungsform der vorliegenden Erfindung, wenn ein erster Fütterungspuls 20-39 Stunden nach Beginn der Kultivierung und ein zweiter Fütterungspuls 40-70 Stunden gegeben wird, wobei es besonders bevorzugt wird, wenn ein erster Fütterungspuls nach 25-35 Stunden und ein zweiter Fütterungspuls nach 50-60 Stunden gegeben wird.

Eine weitere, besonders bevorzugte Ausführungsform der vorliegenden Erfindung besteht in einem kontinuierlichen Zusetzen des Acetats über den gesamten Zeitraum der Kultivierung, wobei das Zusetzen 20 Stunden nach Beginn der Kultivierung, besonders bevorzugt 25 Stunde nach Beginn der Kultivierung begonnen wird, wobei das Acetat in einer Rate von mindestens 0,05 g/L pro Stunde zugesetzt wird.

Bevorzugt handelt es sich bei den GLA-Vorläufermolekülen um Palmitinsäure (C16:0), Stearinsäure (C 18:0), Ölsäure (C18:1) und/oder Linolsäure (C18:2) bzw. um Triglyceride, Diglyceride, Monoglyceride, Phospholipide, Ester oder Salze der entsprechenden Vorläufermoleküle.

In einer bevorzugten Ausführungsform der vorliegenden Erfindung werden die GLA-Vorläufermoleküle in Form von natürlichen Ölen wie Sonnenblumenöl der Kultur zugesetzt.

In einer weiteren bevorzugten Ausführungsform der vorliegenden Erfindung werden die GLA-Vorläufermoleküle in Form von Distelöl der Kultur zugesetzt.

In noch einer weiteren bevorzugten Ausführungsform der vorliegenden Erfindung werden die GLA-Vorläufermoleküle in Form von Olivenöl der Kultur zugesetzt.

Das Acetat wird bevorzugt in Form der Essigsäure oder als Salz der Essigsäure zugesetzt, beispielsweise als Natriumacetat.

In den Untersuchungen der vorstehend genannten Erfinder, die zu vorliegender Erfindung geführt haben, wurde überraschenderweise gefunden, daß eine Zugabe von Acetat vor der Animpfung und auch während der Kultur zu einem schlechteren Wachstum führt (Beispiel 3). Sowohl das Fettsäurespektrum als auch der GLA-Gehalt der Biomasse änderte sich nicht wesentlich, so daß es insgesamt zu einer Verschlechterung der GLA-Ausbeute kommt.

In verschiedenen, daraufhin durchgeführten Fütterungs-Experimenten, bei denen *Tetrahymena* entweder nur Fettsäurevorläufer, nur Triacylglyceriden der Vorläufermoleküle, nur Acetat oder eine Kombination aus Triacylglyceriden der Vorläufermolekille und Acetat gefüttert wurde, wurde überraschenderweise gefunden, daß eine kombinierte Fütterung von *Tetrahymena spec.* mit den genannten Vorläufermolekülen und mit Acetat eine besonders wünschenswerte Steigerung sowohl des Neutrallipid-Gehalts als auch des GLA-Gehaltes in den Neutrallipiden hervorbringt, was zu einer Erhöhung des GLA-Gehaltes in der BTM um 250% von 2,0 % (w/w) auf 5,0 % (w/w) führt. Besonders überraschend war es, daß eine zeitversetzte Zugabe von Acetat, etwa 20-40 Stunden nach Beginn der Kultivierung besonders hohe Ausbeuten an GLA hervorbringt. Zum Vergleich wurden Standardkultivierungen ohne Zugaben von Fettsäurevorläufer und Acetat durchgeführt.

Erfindungsgemäß werden also Ciliaten unter Bedingungen kultiviert, unter denen sie einen hohen PUFA-Gehalt, bevorzugt einen hohen GLA-Gehalt aufweisen.

Unter hohem PUFA-Gehalt bzw. GLA-Gehalt wird dabei für die Zwecke der vorliegenden Erfindung verstanden, daß die entsprechenden Fettsäuren in Form der freien Fettsäuren, als Phospholipid, als Neutrallipid oder auch in anderen Formen, in denen Fettsäuren unter biologischen Normalbedingungen vorliegen können, in der Zelle und/oder der Kulturbrühe vorliegen.

Die Methoden, mit denen das γ-Linolensäurehaltige Lipid bzw. die γ-Linolensäure dann aus der Kultur bzw. der Kulturbrühe isoliert werden können, sind dem Fachmann dabei geläufig.

Ein Verfahren kann es dabei sein, zur Isolierung des γ-Linolensäurehaltigen Lipids aus den Ciliaten folgende Stufen durchzuführen:
(i) Herunterkühlung der Fermentationsbrühe auf 0-10°C, anschließende Zentrifugation oder Filtration der abgekühlten Kulturbrühe,
(ii) Trocknung der erhaltenen Biomasse,
(iii) Extraktion der enthaltenen Lipide, indem man
   (a) die aus (ii) erhaltenen Biotrockenmasse (BTM) einer Extraktion mit einen polaren Lösungsmittel, bevorzugt Ethanol, unterzieht, wodurch das Gesamtlipid extrahiert werden kann.
   (b) anstelle dessen mit einem unpolaren Lösungsmittel, bevorzugt n-Hexan, das Neutrallipid aus der BTM extrahiert, und/oder
   (c) das aus (a) gewonnene Gesamtlipid mit einem unpolaren Lösungsmittel zur Trennung der Neutrallipide und polaren Lipiden, z.B. der Phospholipide, behandelt

Ein weiteres Verfahren zur Isolierung der freien γ-Linolensäure oder deren Ester aus den Ciliaten könnte es sein, folgende Stufen durchzuführen:
(i) Verseifung oder Veresterung der aus den Stufen (ii) oder (iii) des obigen Verfahrens erhaltenen Produkte zur Herstellung der freien Fettsäuren oder Ester
(ii) Anreicherung der GLA durch Fällungsmethoden (z.B. Harnstofffällung) und/oder chromatographische Methoden

Ein weiteres Verfahren zur Isolierung der freien γ-Linolensäure direkt aus der Fermentationsbrühe könnte folgende Stufen umfassen:
(i) Herunterkühlung der Fermentationsbrühe auf 0-10°C, anschließende Zentrifugation oder Filtration (mit anschliessender Verdünnung um eine Fließfähigkeit zu gewährleisten)
(ii) Hydrolyse der freigesetzten Lipide durch Tetrahymena-eigene Lipasen oder durch Zusetzen von anderen Lipasen
(iii) Anreicherung der GLA durch Fällungsmethoden (z.B. Harnstofffällung) und/oder chromatographische Methoden

Weiterhin ist es für den Fachmann naheliegend, aus den z.B. wie oben gezeigt erhaltenen Lipiden, Fettsäuren oder Fettsäureestern Strukturlipide (z.B. Triglyceride, Phospholipide) zu synthetisieren.

Es ist klar, daß dem Fachmann eine Vielzahl ähnlicher Reinigungsmethoden zur Verfügung stehen, die in einem oder mehreren dieser Stufen abgewandelt werden können, ohne daß der Umfang der vorliegenden Erfindung verlassen wird. Selbst ein völlig anderes Reinigungsschema würde hieran nichts ändern, da aus dem Stand der Technik viele unterschiedliche Reinigungsschemata herleitbar sind, und eine solche selektive Aufreinigung dem Standardkönnen des Fachmanns entspricht.

Die nachfolgend erläuterten Figuren tragen zum besseren Verständnis der vorliegenden Erfindung bei.
- **Figur 1**: zeigt ein allgemeines Schema der Biosynthese von PUFA in Eukaryoten
- **Figur 2**: zeigt die erreichte Biotrockenmasse in g/L unter Verwendung von MM-Var (Standardmedium), MM-Var + Distelöl bzw. MM-Var + Olivenöl (je zwei Parallelversuche)
- **Figur 3**: zeigt die erreichten Gewichtsprozent GLA bezogen auf die Biotrockenmasse in % unter Verwendung von MM-Var (Standardmedium), MM-Var + Distelöl bzw. MM-Var + Olivenöl (je zwei Parallelversuche)
- **Figur 4**: zeigt die erreichte GLA-Ausbeute in mg/L unter Verwendung von MM-Var (Standardmedium), MM-Var + Distelöl bzw. MM-Var + Olivenöl (je zwei Parallelversuche)
- **Figur 5**: zeigt die erreichte GLA-Ausbeute in Area[%] in g/L unter Verwendung von MM-Var (Standardmedium), MM-Var + Distelöl bzw. MM-Var + Olivenöl (je zwei Parallelversuche). Die Area[%] zeigen den Anteil der GLA an dem Gesamtlipidgehalt.
- **Figur 6**: zeigt den Verlauf der Zellzahl bei Zugabe von NaAc ins MM-VAR Grundmedium
- **Figur 7**: zeigt den Verlauf der BTM bei Zugabe von NaAc ins MM-Var Grundmedium
- **Figur 8**: zeigt den GLA-Gehalt der BTM bei Zugabe von NaAc ins MM-Var Grundmedium
- **Figur 9**: zeigt die GLA-Konzentration der Kultur bei Zugabe von NaAc ins MM-Var Grundmedium
- **Figur 10**: zeigt den Verlauf der Zellzahl bei Feeding von NaAc während der Kultivierung (t = 25 h, 54 h); die Pfeile kennzeichnen den Zeitpunkt der Zugabe
- **Figur 11**: zeigt den Verlauf der BTM bei Feeding von NaAc während der Kultivierung (t=25 h, 54 h); die Pfeile kennzeichnen den Zeitpunkt der Zugabe
- **Figur 12**: zeigt den GLA-Gehalt bei Feeding von NaAc während der Kultivierung (t=25h, 54 h)
- **Figur 13**: zeigt die GLA-Konzentration der kulturbei Feeding von NaAc während der Kultivierung (t = 25 h, 54 h)
- **Figur 14**: zeigt den Einfluß von 0,5 g/L NaAc und 0,1 % Distelöl auf die Zellzahl von *T. thermophila* B1868VII.
- **Figur 15**: zeigt den Einfluß von 0,5 g/L NaAc und 0,1 % Distelöl auf die BTM von *T. thermophila* B1868VII.
- **Figur 16**: zeigt den Verlauf des GLA-Gehaltes von *T. thermophila* B1868VII bei Wachstum auf 0,5 g/L NaAc und 0,1 % Distelöl
- **Figur 17**: zeigt den Einfluß von 0,5 g/L NaAc und 0,1 % Distelöl auf die GLA-Ausbeute während einer Kultivierung von *T. thermophila* B1868VII
- **Figur 18**: zeigt den Verlauf der GLA-Area in der BTM von von *T. thermophila* B1868VII bei Wachstum auf 0,5 g/L NaAc und 0,1 % Distelöl

Dem Fachmann ist angesichts der vorstehenden Ausführungen klar, das mit einer Vielzahl weiterer *Tetrahymena* spec., wie z.B. *T*. *silvana (z. B. ATCC 50084), T. malaccensis (z. B. ATCC 50065), T. asiatica (z. B. ATCC 50068), T. borealis (z. B. ATCC 30203), T. canadensis (z. B. ATCC 50098), T. australis (z. B. ATCC 30349), T. vorax (z. B. ATCC 30421), T. furgasoni (z. B. ATCC 9357), T. setosa (z. B. ATCC 30782) und T. tropicalis (z. B. ATCC 30275)* ähnlich gute GLA-Ausbeuten unter Anwendung des erfindungsgemäßen Verfahrens erreicht werden können.

Die im folgenden aufgeführten Beispiele erläutern die Erfindung näher. Die Beispiele sollen jedoch nicht einschränkend verstanden werden. Wenn nicht anders angegeben wurde, wurden für die einzelne Versuche *Tetrahymena thermophila* B1868VII (z. B. ATCC 30384) verwendet.

### BEISPIEL 1

### Standardkultivierung

20 mL MM-Var (siehe Tabelle 1) Medium wurden in 100 mL Erlenmeyerkolben bzw. 100 mL MM-Var Medium in 500 mL Erlenmeyerkolben mit Schikanen gefüllt, mit einem Zellstoff/Baumwoll-Stopfen verschlossen und für 20 min. bei 121 °C sterilisiert.

Gegebenfalls wurden vor dem Sterilisieren weitere Zusätze in das Medium gegeben.

**Tabelle 1 Zusammensetzung MM-Var Medium**

| **Substrat** | **Konzentration** |
|---|---|
| **Magermilchextrakt** | 2 % |
| Glucose | 1 % |
| Hefeextrakt | 0,5 % |
| Na-Fe-EDTA | 0,003 |
| Bestandteile werden in Leitungswasser gelöst | |

### Vorkulturen

Für die Vorkulturen wurden 100 mL-Kolben mit 20 mL Medium mit MM-var Medium (siehe Tabelle 1) einer Zelldichte von ca. 5x10⁴ - 1x10⁵ Zellen pro mL angeimpft und über 2 Tage bei 25°C, 60 rpm kultiviert. Die Zellzahl erreichte einen Wert zwischen 1x10⁶ und 3x10⁶ Zellen pro mL.

### Hauptkulturen in Schüttelkolben

Die Schüttelkolbenversuche würden in 500 mL Erlenmeyer mit 100 mL Medium durchgeführt. Die Versuchskolben wurden mit einer Zelldichte von ca. 5x10⁴-1x10⁵ Zellen pro mL angeimpft und über 2 Tage bei 25°C, 100 rpm kultiviert.

### Fermentationen im 1-L bzw. 5-L Maßstab

Die Fermentationen wurden in 1-L Fermenter (700 mL Arbeitsvolumen), 5-L Fermenter (3 L Arbeitsvolumen) bzw. 13-L Fermenter (10 L Arbeitsvolumen) durchgeführt. Standardmäßig waren die Fermenter mit einer pH-Elektrode, einer pO₂-Elektrode, einen Belüftungsring, einer Rührwelle mit Scheibenblattrührer, Zuleitung für Säure/Lauge, Zuleitung für Feeding, Animpfarmatur und eine Probenahme versehen.

Die Fermenter wurden mit dem Arbeitsvolumen an Medium gefüllt und in einem Sterilisator für eine halbe Stunde bei 123°C sterilisiert,

Die Fermentionsbedingungen waren wie in Tabelle 2 angegeben.

**Tabelle 2 Prozeßparameter**

| **Parameter** | **Wert** |
|---|---|
| Belüftungsrate | 1vvm |
| pH | 7 , reguliert mit Säure (z.B. Phosphorsäure) und Base (z.B. NaOH) |
| Rührerspitzengeschwindig keit | Linearer Anstieg von 0,3 m/s auf 1,2 m/s in den ersten 24 Stunden |
| Temperatur | 28°C |
| Animpfdichte | 5x10⁴-1x10⁵ Zellen pro mL |

Angeimpft wurden die Fermentationen mit einer Hauptkultur aus einer Schüttelkultur (siehe oben).

### Bestimmung der Biomasse

Für die Bestimmung der Biotrockenmasse wurden 5 mL Zellkultur für 10 min bei 3400 g und 4°C zentrifugiert, der Überstand dekantiert und das Zellpellet bei -80°C eingefroren. Die gefrorene Biomasse wurde lyophilisiert und das Gewicht der Biotrockenmasse bestimmt.

### Bestimmung der Zellzahl

Die Zelldichte wurde mit dem CASY 1 Cell Counter + Analyser System, Model TTC, Schärfe System ermittelt.
Für eine Messung wurden die Zellen in einer schwachen CASY-Elektrolytlösung verdünnt (1:500) und durch eine Kapillare mit 150 µm Durchmesser gefördert. Die Zellzählung erfolgte in einem Bereich von 15-50 µm Partikelgröße.

### Bestimmung des Neutrallipidgehaltes in der Biotrockenmasse

Die unpolaren Lipidbestandteile (Neutrallipide) wurden mit n-Hexan aus der Biotrockenmasse extrahiert. Für einen Ansatz wurden ca. 500 mg Biotrockenmasse in 5 mL n-Hexan für 1 h bei 60°C gerührt, anschließend 2 min bei 2700 g zentrifugiert und der Überstand durch Filtration von Schwebstoffen gereinigt. Für die Filtration wurde eine mit Watte gefüllte Pasteur-Pipette verwendet, mit einer Füllhöhe von ca. 1 cm. Der Zellrückstand aus der ersten Extraktion wurde ein weiteres mal mit 5 mL Lösungsmittel extrahiert, die Biomasse abzentrifugiert und der Überstand filtriert. Die gereinigten, organischen Phasen der beiden Extraktionen wurden vereint, das Lösungsmittel am Rotationverdampfer vollständig verdampft und die Menge des extrahierten Lipids durch Wägung bestimmt.

### Qualitative und quantitative Analyse extrahierter Lipide

Eine qualitative und quantitative Analyse extrahierter Lipide erfolgte mit Hilfe des IatroscanTM new MK-5-Analysers. Als Chromatographiematerial dienten Trennstäbe (Chromarods) mit thermoresistenten Silicagel als Oberflächenmaterial. Die Stäbe wurden in einem Exikator gelagert und vor jedem Trennvorgang zur Entfernung störender Substanzen abgebrannt. Als Meßbedingungen des FID wurden gewählt: Scanrate 30 s/scan, O₂-Strom 2L/min und H₂-Strom 160 mL/min.

Das zu analysierende Lipid wurde in einer Konzentration von ca. 10 g/L in Chloroform/ Methanol (2: 1) gelöst und davon 1 µL auf einen Trennstab aufgetragen. Die Trennung erfolgte über einen Zeitraum von 30 min in einem Laufmittel, dessen Zusammensetzung in Tabelle 3 angegeben ist.

**Tabelle 3 Laufmittel für die Iatroscan-Analyse von Lipiden**

| **Laufmittel** | **Zusammensetzung** | **Verhältnis (v/v)** |
|---|---|---|
| zur Trennung von Neutrallipiden | *n*-Hexan, Chloroform, Isopropanol, Formiat, Acetat | 800 : 160 : 10 : 1 : 10 |

Nach dem Lauf wurden die Stäbe 5 min bei 80°C getrocknet und im Iatroscan analysiert. Die Zuordnung und Quantifizierung der Lipide erfolgte durch einen Vergleich mit authentischen Standards. Als Standardlösung wurde ein Gemisch aus 4,65 g/L Tripalmitinsäure, 5,17 g/L Dipalmitinsäure und 5,64 g/L Palmitinsäure gelöst in Methanol/Chloroform (2:1) verwendet. Zur Erstellung von Kalibriergeraden wurde die Lösung in Mengen von 0,5-2,0 µL auf die Trennstäbe aufgetragen, in Lösungsmittel entwickelt und am DC-FID analysiert (Tabelle 4).

**Tabelle 4 Quantifizierung von Fettsäuren anhand der Ergebnisse einer GC-Analyse**

| **Angabe** | **Definition** | **Abkürzung** | **Einheit** |
|---|---|---|---|
| Area % | rel. Anteil einer FS an den Gesamtfettsäuren | FS/GFS | Area % |
| Massenprozent (w/w) | abs. Anteil einer FS am NL oder an der BTM (berechnet über ISTD) | FS/NL, FS/BTM | % |

### Bestimmung des GLA-Gehaltes mittels Gaschromatographie (GC)

Die Bestimmung des Fettsäurespektrums erfolgte mittels Gaschromatographen (HP GC 6890) mit Flammenionisationsdetektor (Hewlett-Packard Company, Wilmington, USA). Als Säule diente eine FFAP (Free Fatty Acid Phase) Permbond (Macherey & Nagel GmbH, Düren). Die Identifizierung der Fettsäuren erfolgte durch den Vergleich mit Retentionszeiten von Fettsäuremethylester-Standards. Auf der Basis der bekannten Konzentration des Standards konnte die Konzentration der Fettsäuren in den Proben bestimmt werden.

30-70 mg der lyophillisierten Biotrockenmasse (siehe Bestimmung der Biomasse) wurden eingewogen und mit 1 mL 20%iger methanolischer HCl und 1 mL methanolischer Standard-Lösung (1 mg/mL) versetzt. Zur Freisetzung der Fettsäuren und deren Umesterung in Fettsäuremethylester wurden die Proben im geschlossenen Reagenzglas zwei Stunden bei 60°C im Wasserbad gerührt und auf Raumtemperatur abgekühlt. Zum Neutralisieren der Probe wurde anschließend 1 mL wässrige, gesättigte Natriumhydrogencarbonat-Lösung zugegeben und vorsichtig gemischt. Durch Zugabe von n-Hexan wurden die Fettsäuremethylester extrahiert. Anschließende wurde der Ansatz kräftig durchmischt und durch Zentrifugation für 2 min. bei 4300 rpm eine Phasentrennung erreicht. Etwa 2/3 der oberen, organischen Phase wurden entnommen und 1 µl der Probe wurde auf die GC-Säule gespritz und analysiert (s. auch Tabelle 5).

**Tabelle 5 Meßbedingungen des HP 6890 Gaschromatographen**

| | |
|---|---|
| Trägergas: Helium | 1,0 mL/ min |
| Make up: Helium | 20 mL/ min |
| Injektortemperatur: | 255°C |
| Splitbetrieb | 10:1 |
| Injektionsvolumen | 1 µL |
| Detektortemperatur: | 255°C |
| FID-Brenngase | Wasserstoff (30 mL/min), Sauerstoff (300 mL/min) |
| Temperaturprofil | 160°C, 12 min -10°C/min bis 230°C - 230°C, 5 min |

### BEISPIEL 2

### Effekt von Distelöl (Linolsäurequelle) auf die GLA-Produktion

Zu zwei 500 mL Kolben (100 mL MM-Var Medium wurde 1 mL/L Distelöl vor der Sterilisation zugesetzt. Als Referenz dienten zwei 500 mL Kolben (100 mL MM-Var Medium. Das Distelöl hatte die in Tabelle 6 angegebene Zusammensetzung. Die Fettsäuren lagen in Form der Triglyceride vor.

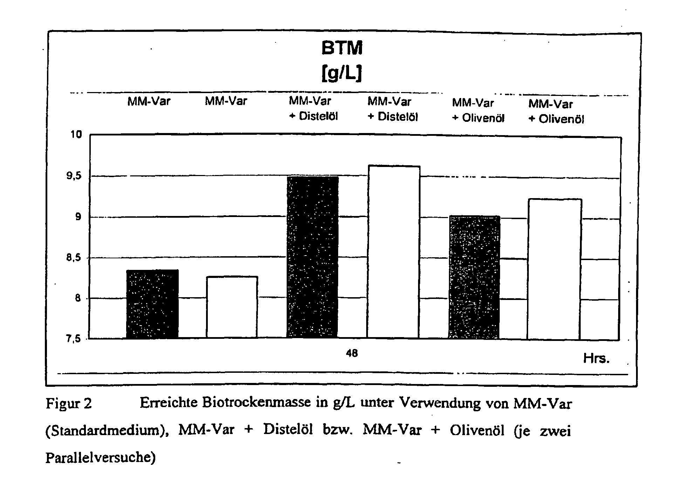

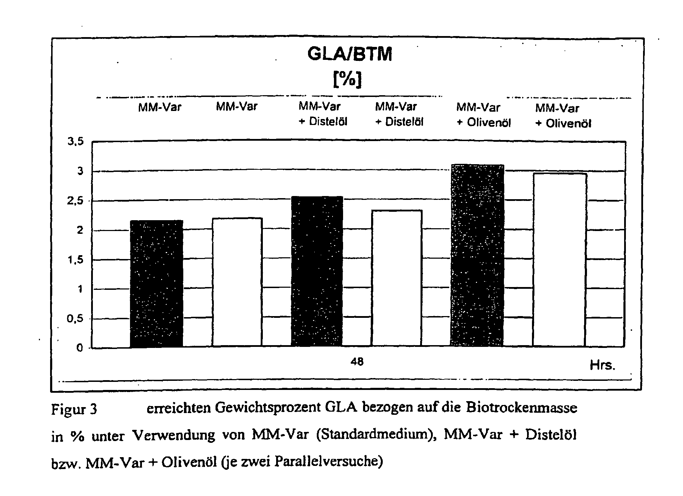

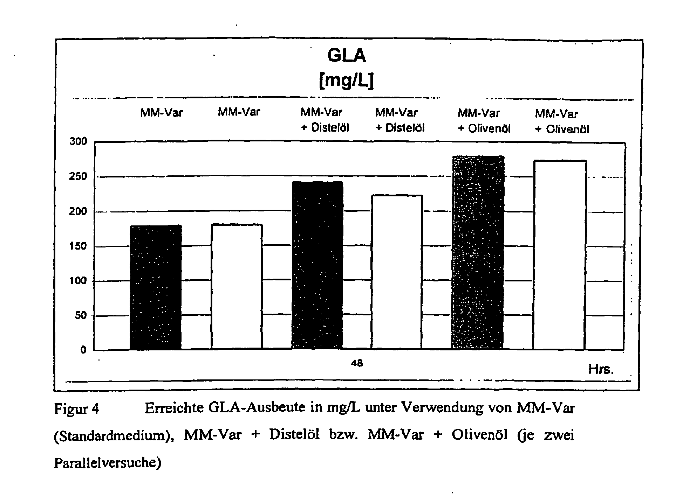

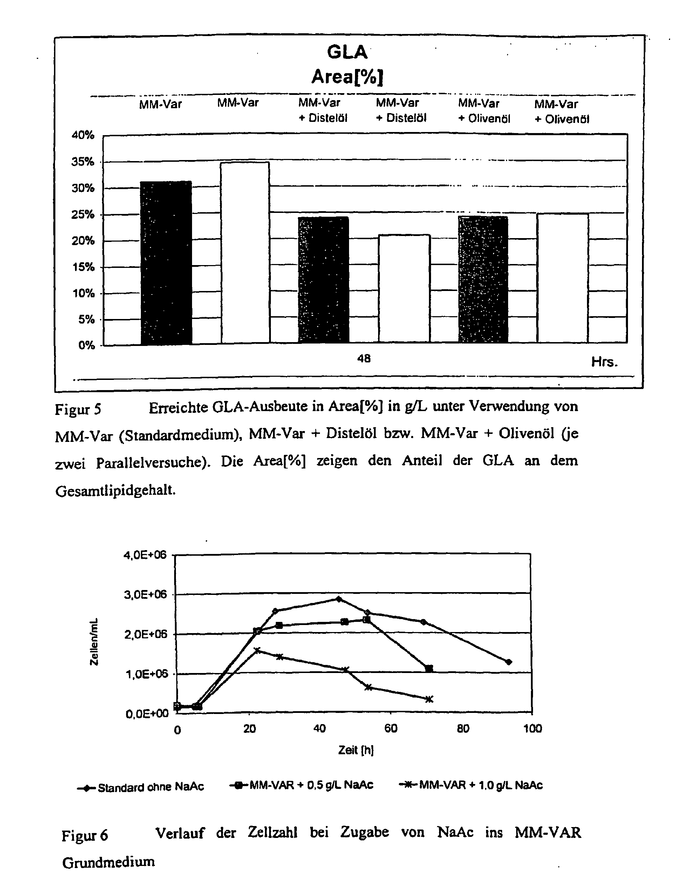

Die Ergebnisse sind in Figur 2 bis Figur 4 dargestellt. Durch die Zugabe konnte eine Biomassesteigerung von ca. 8,3 g/L auf ca. 9,5 g/L erreicht werden (Figur 2). Der GLA-Gehalt in der Biomasse wurde nur geringfügig erhöht (Figur 3), aber durch die höhere Biomassekonzentration konnte die volumetrische GLA-Konzentration um 28% (von 180 mg/L auf 230 mg/L) gesteigert werden. Durch die Zugabe des Distelöls wurde der GLA-Anteil in den Gesamtfettsäuren von 33% auf 22% erniedrigt (Figur 5).

**Tabelle 6 Typische Fettsäuregehälter verschiedener Naturgrundstoffe**

| **Naturstoff** | **Stearinsäure** | **Ölsäure** | **Linolsäure** | **γ-Linolensäure** |
|---|---|---|---|---|
| Distelöl/Safloröl | 2,5% | 12,5% | 78,2% | |
| Nachtkerzenöl | 2% | 8,8% | 73,8% | 8,3 |
| Olivenöl | 2,5% | 75,5% | 8,4% | |
| Sonnen-blumenöl | 4% | 25% | 65% | |

### BEISPIEL 3

### Effekt von Olivenöl (Ölsäurequelle) auf die GLA-Produktion

Zu zwei 500 mL Kolben (100 mL MM-Var Medium wurde 1 mL/L Olivenöl vor der Sterilisation zugesetzt. Als Referenz dienten zwei 500 mL Kolben (100 mLMM-Var Medium. Das Olivenöl hatte die in Tabelle 6 (oben) angegebene Zusammensetzung. Die Fettsäuren lagen in Form der Triglyceride vor.
Die Ergebnisse sind in Figur 2 bis Figur 4 dargestellt. Durch die Zugabe konnte eine Biomassesteigerung von ca. 8,3 g/L auf ca. 9,1 g/L erreicht werden (Figur 2). Der GLA-Gehalt in der Biomasse wurde signifikant erhöht (von 2,1 % auf 3%) (Figur 3), und in Zusammenhang mit der höheren Biomassekonzentration konnte die volumetrische GLA-Konzentration um 53% (von 180 mg/L auf 275 mg/L) gesteigert werden. Durch die Zugabe des Olivenöls wurde der GLA-Anteil in den Gesamtfettsäuren von 33% auf 24% erniedrigt (Figur 5).

### BEISPIEL 4

### Effekt von Acetat auf die GLA-Produktion (Acetat im Grundmedium)

Die Fermentationen wurden in 1-L Fermenter mit 700 mL MM-Var Medium durchgeführt. Eine Fermentation diente als Referenz, zu den anderen beiden wurden 0,5 bzw. 1,0 g/L Natriumacetat (NaAc) dem Kulturmedium vor dem Sterilisieren zugegeben.

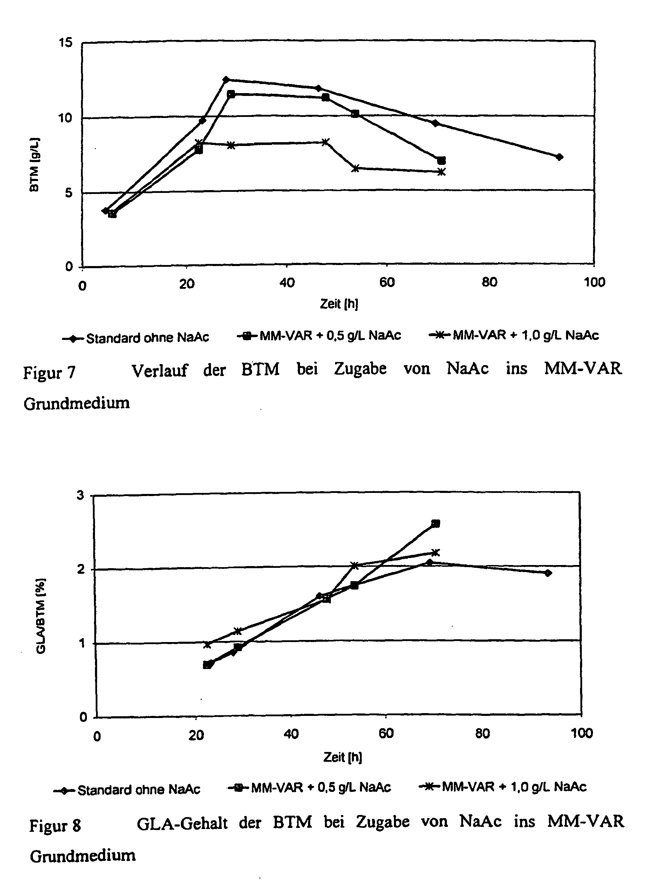

Natriumacetat wirkte sich bei den Kultivierungen negativ auf das Zellwachstum von *Tetrahymena thermophila* B1868VII aus (Figuren 6 und 7). Sowohl die Zelldichte als auch die Biotrockenmasse (BTM) der Experimente lagen unterhalb der Ergebnisse der Referenz-Fermentation. Dabei bestand ein direkter Zusammenhang zwischen eingesetzter Menge an Natriumacetat und der inhibierenden Wirkung auf das Zellwachstum. Der GLA-Gehalt der Biotrockenmasse wurde durch die Zugabe von NaAc nicht verändert (Figur 8).

Auch die Synthese von Neutrallipiden wurde durch die Zugabe von Natriumacetat nur gering verändert (s. Tabelle 7).

**Tabelle 1 Lipid-Gehalt bei Zugabe von NaAc in das Grundmedium**

| Fermentation | NL/BTM | [% (w/w)] | GLA/NL | [% (w/w)] | GLA/NL | [Area %] |
|---|---|---|---|---|---|---|
| | 46 h | 70 h | 46 h | 70 h | 46 h | 70 h |
| Standard ohne NaAc | 8 | 11 | 8 | 10 | 17 | 26 |
| MM-VAR mit 0,5 g/L NaAc | 8 | 17 | 10 | 12 | 18 | 22 |
| MM-VAR mit 1,0 g/L NaAc | 11 | 17 | 8 | 14 | 16 | 26 |

Nach einer Kultivierungszeit von 46 h war noch kein signifikanter Unterschied zur Standard-Fermentation zu erkennen. Erst nach 70 h wurde ein Anstieg beobachtet: Die Zugabe von NaAc ins MM-Var Grundmedium führte zu einer Steigerung des NL-Gehalts von 11% auf 17 % (w/w). Der GLA-Gehalt im Neutrallipid zeigte nur geringe Unterschiede zur Standardfermentation.

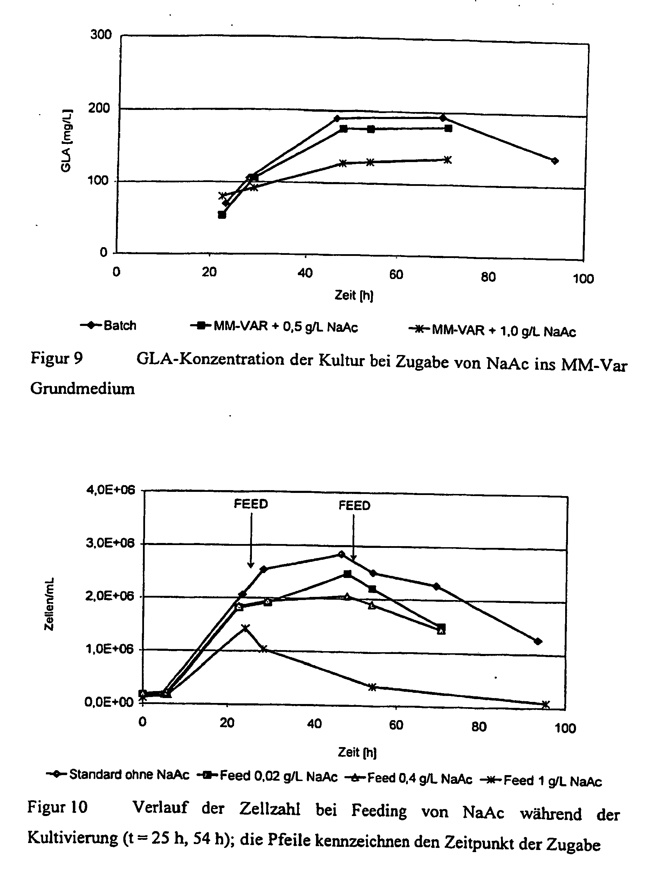

Die volumetrische GLA-Produktbildung verschlechterte sich überraschenderweise durch die Zugabe von NaAc (Figur 9).

### BEISPIEL 5

### Effekt von Acetat auf die GLA-Produktion (Acetat nach 24 Stunden Kultur zugegeben)

Die Fermentationen wurden im 1-L Fermenter mit 700 mL MM-Var Medium durchgeführt. Eine Fermentation diente als Referenz, zu den anderen wurden 0.2, 0.5 bzw. 1.0 g/L Natriumacetat (NaAc) in Form einer sterilisierten (20 min, 121°C), 10%-igen Lösung zu der 29. Fermentationsstunde zugegeben. Bei den Fermentionen, zu denen zur 29. Stunde 0.2 und 0.5 g/L NaAc zugefüttert wurden, wurde dieselbe Menge zu der 54. Fermentationsstunde erneut zugegeben.

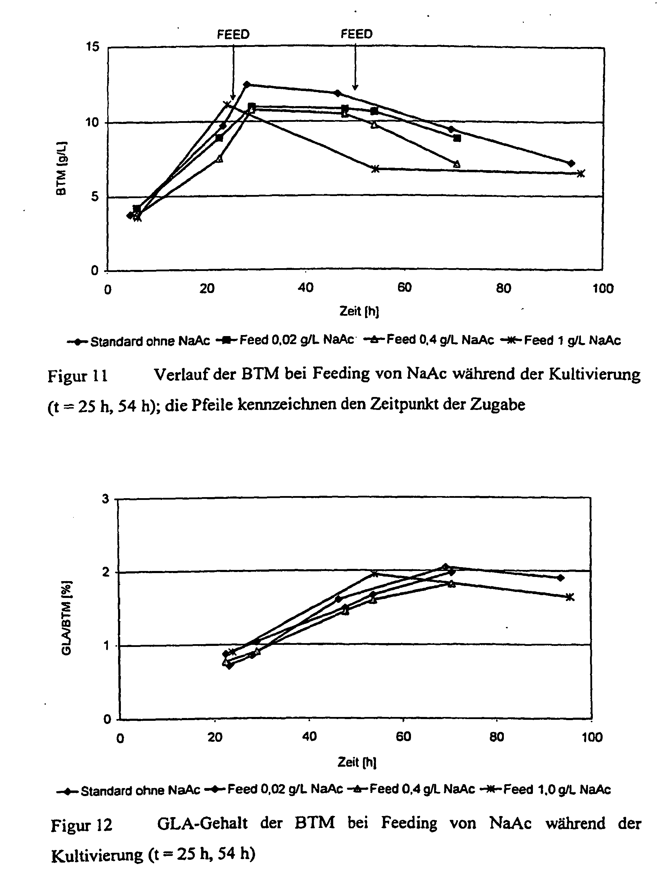

Natriumacetat wirkte sich bei den Kultivierungen negativ auf das Zellwachstum von Tetrahymena thermophila B1868VII aus (Figuren 10 und 11). Sowohl die Zelldichte als auch die BTM der Experimente lagen unterhalb der Ergebnisse der Referenz-Fermentation. Dabei bestand ein direkter Zusammenhang zwischen eingesetzter Menge an Natriumacetat und der inhibierenden Wirkung auf das Zellwachstum. Bei Feeding von NaAc während der Kultivierung ist als direkte Reaktion ein vermindertes Wachstum bzw. ein Absinken der Zelldichte festzustellen. Der GLA-Gehalt der Biotrockenmasse wurde durch die Zugabe von NaAc nicht verändert (Figur 12).

Auch die Synthese von Neutrallipiden wurde durch die Zugabe von Natriumacetat nur gering verändert (s. Tabelle 8).

**Tabelle 2 Lipid-Gehalt bei Feeding von NaAc**

| Fermentation | NL/BTM | [% (w/w)] | GLA/NL | [% (w/w)] | GLA/NL | [Area %] |
|---|---|---|---|---|---|---|
| | 46 h | 70 h | 46 h | 70 h | 46 h | 70 h |
| Standard ohne NaAc | 8 | 11 | 8 | 10 | 17 | 26 |
| Feed 0,02 g/L NaAc | 8 | 12 | 8 | 14 | 15 | 26 |
| Feed 0,4 g/L NaAc | 9 | 13 | 7 | 11 | 15 | 20 |

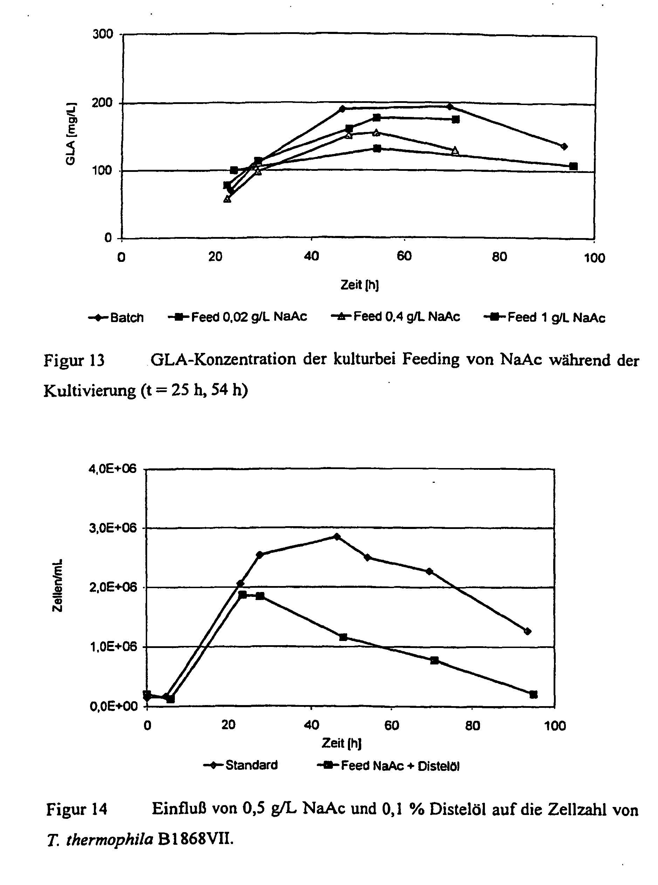

Nach einer Kultivierungszeit von 46 h auf Acetat-Medium war kein Unterschied zur Standard-Fermentation zu erkennen. Auch nach 70 h wurde kein signifikanter Anstieg beobachtet.
Die volumetrische GLA-Produktbildung verschlechterte sich durch die Zugabe von Acetat (Figur 13).

### BEISPIEL 6

### Feeding von Natriumacetat und Distelöl

Die Fermentationen wurden in 1-L Fermenter mit 700 mL MM-Var Medium durchgeführt. Eine Fermentation diente als Referenz, zu einer wurden 0.5 g/L NaAc und 1 mL/L Distelöl vor dem Sterilisieren zugesetzt und zu einem wurden 0.5 g/L NaAc und 1 mL/L Distelöl gegen Ende der exponentiellen Phase (zur 28. Stunde) zugesetzt.

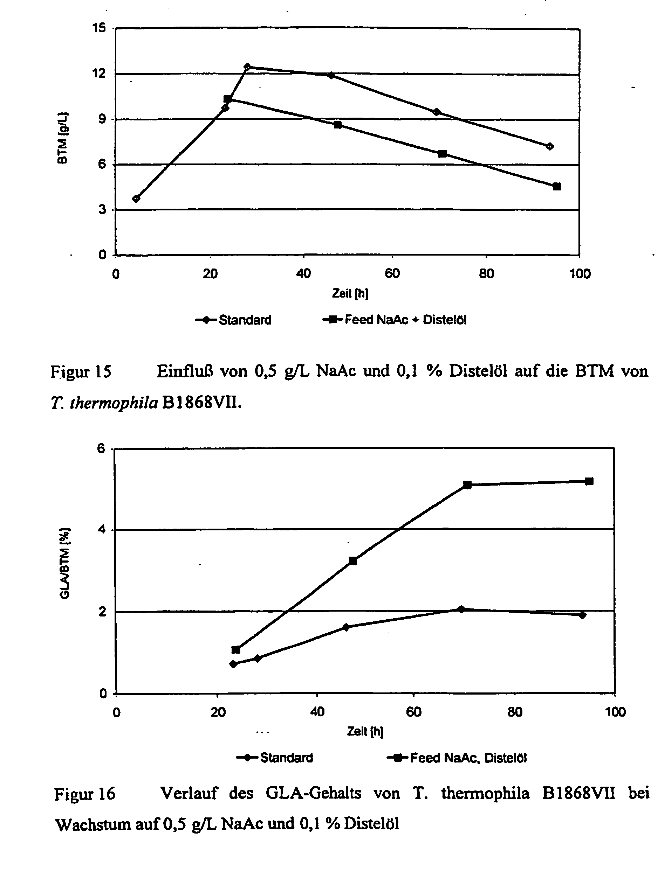

In Figur 14 und Figur 15 ist der Verlauf der Zellzahl und Biotrockenmasse dargestellt. Es ist zu erkennen, daß die Zugabe von NaAc und Distelöl ins Kulturmedium zu einem verminderten Wachstum von *Tetrahymena* führte. Bei Substratzugabe zu Fermentationsbeginn wurde im Vergleich zur Referenz-Fermentation eine verzögerte exponentielle Wachstumsphase beobachtet; die erreichte BTM war annähernd gleich. Eine Zugabe nach 28 h führte zu einer Abnahme der BTM infolge der Fütterung.

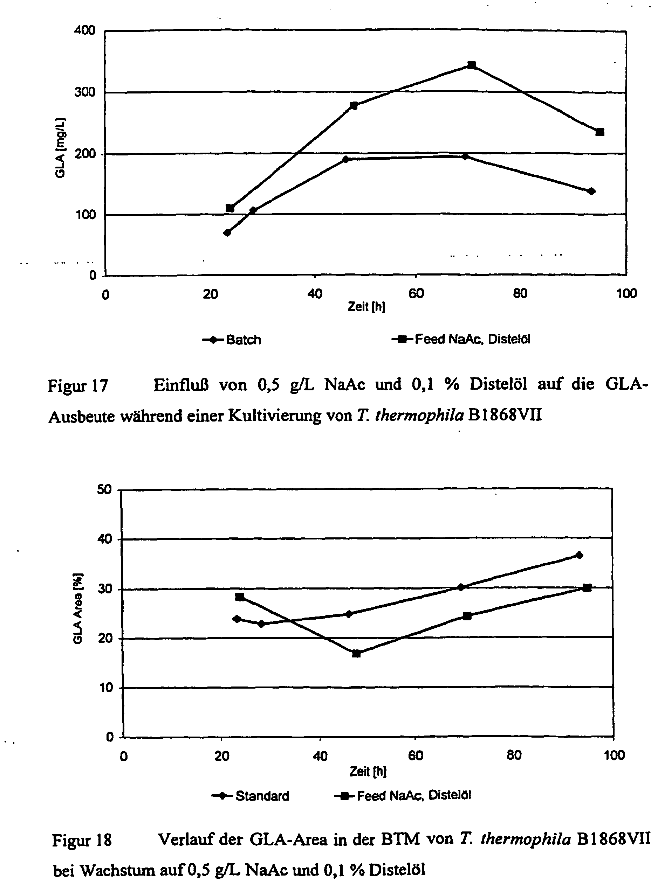

In Figur 16 ist der GLA-Gehalt pro Biomasse im Verlauf der Fermentation aufgetragen. Die Kultivierung auf Distelöl und Natriumacetat bewirkte eine starke Erhöhung des GLA-Gehalts auf 5,2 % (w/w). Die Standard-Fermentation zeigte deutlich geringe Werte von 2-3 % (w/w). Der erhöhte GLA-Gehalt drückte sich trotz einer verminderten BTM in einer hohen GLA-Konzentration von 342 mg/L (71 h; Figur 17) mit einer Reinheit der GLA von 30 Area% (Figur 18) aus.

### BEISPIEL 7

### Kombinierte Zufütterung Olivenöl und Acetat

Ähnlich gute Ergebnisse konnten auch durch Verwendung von Olivenöl erzielt werden.

## Patentansprüche

1. Verfahren zur fermentativen Herstellung von omega-6-Fettsäuren, insbesondere γ-Linolensäure, **dadurch gekennzeichnet, dass** man Ciliaten in einem Kulturmedium kultiviert, dem γ-Limolensäure-Vorlänfermoleküle und Acetat zugesetzt werden, wobei das Acetat in zwei Stufen zugesetzt wird, wobei ein erster Zusatz 20-39 Stunden und ein zweiter Zusatz 40-70 Stunden nach Beginn der Kultur erfolgt, und die γ-Linolensäure nach Beendigung der Kultur aus den Ciliaten isoliert wird.

2. Verfahren nach Anspruch 1, **dadurch gekennzeichnet, dass** *Tetrahymena spec.,* eingesetzt wird.

3. Verfahren nach Anspruch 2, **dadurch gekennzeichnet, dass** *Tetrahymena thermophila* eingesetzt wird.

4. Verfahren nach Anspruch 2, **dadurch gekennzeichnet, dass** *Tetrahymena pyriformis* eingesetzt wird.

5. Verfahren nach Anspruch 2, **dadurch gekennzeichnet, dass** *Tetrahymena rostrata* eingesetzt wird.

6. Verfahren nach einem der vorstehenden Ansprüche, **dadurch gekennzeichnet, dass** das Acetat 25-35 Stunden nach Beginn der Kultur zugesetzt wird.

7. Verfahren nach einem der vorstehenden Ansprüche, **dadurch gekennzeichnet, dass** das Acetat in zwei Stufen zugesetzt wird, wobei ein erster Zusatz 25-35 Stunden und ein zweiter Zusatz 50-60 Stunden nach Beginn der Kultur erfolgt.

8. Verfahren nach einem der vorstehenden Ansprüche, **dadurch gekennzeichnet, dass** die γ-Linolensäure-Vorläufermoleküle in Form von Palmitinsäure (C16:0), Stearinsäure (C18:0), Ölsäure (C18:1) und/oder Linolsäure (C18:2) bzw. in Form von Triglyceriden, Diglyceriden, Monoglyceriden. Phospholipiden, Estern oder Salzen
der entsprechenden Fettsäurevorläufer, ganz besonders bevorzugt in Form von natürlichen Ölen wie Distelöl Olivenöl oder Sonnenblumenöl der Kultur zugesetzt werden.

9. Verfahren nach einem der vorstehenden Ansprüche, **dadurch gekennzeichnet, dass** das Acetat in Form von Essigsäure oder als Salz der Essigsäure der Kultur zugesetzt wird.

10. Verfahren nach Anspruch 8, **dadurch gekennzeichnet, dass** das Pflanzenöl in einer Endkonzentration von 0,1 mL/L bis 10 mL/L dem Medium zugesetzt wird.

11. Verfahren nach einem der vorstehenden Anspruch, **dadurch gekennzeichnet, dass** das Acetat als Natriumacetat in einer Konzentration von 0,1 g/L bis 1g/L zugesetzt wird.

12. Verfahren nach einem der vorstehenden Ansprüche, **dadurch gekennzeichnet, dass** der GLA-Gehalt pro Biotrockenmasse aus der Kultur mindestens 4% (w/w), besonders bevorzugt mindestens 5% (w/w) beträgt.

13. Verfahren nach einem der vorstehenden Ansprüche, **dadurch gekennzeichnet, dass** die γ-Linolensäure-Konzentration in der Kultur mindestens 300 mg/l beträgt.

14. Verfahren nach einen der vorstehenden Ansprüche, **dadurch gekennzeichnet, dass** die Reinheit der γ-Linolensäure bezogen auf den Gesamtlipidgehalt der Kultur mindestens 30 Area% beträgt.

15. Biotrockenmasse aus einer Ciliatenkultur, **dadurch gekennzeichnet, dass** die Biotrockenmasse wenigstens 4% (w/w) γ-Linolensäure enthält.

## Claims

1. A method for the fermentative production of omega-6 fatty acids, in particular γ-linolenic acid, **characterized in that** ciliates are cultivated in a culture medium to which γ-linolenic acid precursor molecules and acetate are added, the acetate being added in two steps wherein a first addition takes place 20-39 hours and a second addition 40-70 hours after starting the culture and the γ-linolenic acid is isolated from the ciliates after completion of the culture.

2. The method according to claim 1, **characterized in that** *Tetrahymena spec.* is used.

3. The method according to claim 2, **characterized in that** *Tetrahymena thermophila* is used.

4. The method according to claim 2, **characterized in that** *Tetrahymena pyriformis* is used.

5. The method according to claim 2, **characterized in that** *Tetrahymena rostrata* is used.

6. The method according to any one of the preceding claims, **characterized in that** the acetate is added 25-35 hours after starting the culture.

7. The method according to any one of the preceding claims, **characterized in that** the acetate is added in two steps wherein a first addition takes place 25-35 hours and a second addition 50-60 hours after starting the culture.

8. The method according to any one of the preceding claims, **characterized in that** the γ-linolenic acid precursor molecules are added to the culture in the form of palmitic acid (C16:0), stearic acid (C18:0), oleic acid (C18:1) and/or linoleic acid (C18:2) or in the form of triglycerides, diglycerides, monoglycerides, phospholipids, esters or salts of the corresponding fatty acid precursors, very particularly preferably in the form of natural oils such as safflower oil, olive oil or sunflower oil.

9. The method according to any one of the preceding claims, **characterized in that** the acetate is added to the culture in the form of acetic acid or as a salt of acetic acid.

10. The method according to claim 8, **characterized in that** the vegetable oil is added to the medium in a final concentration of 0.1 mL/L to 10 mL/L.

11. The method according to any of the preceding claims, **characterized in that** the acetate is added as sodium acetate in a concentration of 0.1 g/L to 1 g/L.

12. The method according to any one of the preceding claims, **characterized in that** the GLA content per biomass dry weight from the culture is at least 4% (w/w), particularly preferably at least 5% (w/w).

13. The method according to any one of the preceding claims, **characterized in that** the γ-linolenic acid concentration in the culture is at least 300 mg/L.

14. The method according to any one of the preceding claims, **characterized in that** the purity of the γ-linolenic acid is at least 30% by area, based on the total lipid content of the culture.

15. A biomass dry weight from a ciliate culture, **characterized in that** the biomass dry weight contains at least 4% (w/w) of γ-linolenic acid.

## Revendications

1. Procédure pour la production des acides gras oméga-6, en particulier des acides linoléiques γ, par fermentation, **caractérisée en ce que** l'on cultive des ciliathes dans un medium de culture, et qu'on ajoute une molécule précurseur d'acide linoléique γ, en ajoutant l'acétate en deux étapes, en faisant un premier ajout 20 - 39 heures après le commencement de la culture et un deuxième ajout 40 - 70 heures après le commencement de la culture et en isolant l'acide linoléique γ après la conclusion de la culture de ciliathes.

2. Procédure selon revendication 1, **caractérisée en ce qu'**on insère la substance *tetraphymena spec.*

3. Procédure selon revendication 2, **caractérisée en ce qu'**on insère la substance *tetraphymena thermophila.*

4. Procédure selon revendication 2, **caractérisée en ce qu'**on insère la substance *tetrapymena pyriformis.*

5. Procédure selon revendication 2, **caractérisée en ce qu'**on insère la substance *tetraphymena rostrata.*

6. Procédure selon l'une des revendications précédentes, **caractérisée en ce que** l'acétate est ajoutée 25 - 35 heures après le commencement de la culture.

7. Procédure selon l'une des revendications précédentes, **caractérisée en ce que** l'acétate est ajoutée en deux étapes, en faisant un premier ajout 25 - 35 heures après le commencement de la culture et un deuxième ajout 40 - 60 heures après le commencement de la culture.

8. Procédure selon l'une des revendications précédentes, **caractérisée en ce que** les molécules précurseurs d'acide linoléique γ sont ajoutées sous forme d'acide de palmitine (C16 :0), d'acide de stéarine (C18 :0), d'acide de huile (C18 :1) et/ou d'acide linoléique (C18:2) et/ou sous forme de triglycérides, di-glycérides, mono-glycérides, phospholipides, esters ou sels des précurseurs d'acides gras, très préférablement sous forme d'huiles naturelles comme l'huile de chardon, l'huile d'olive ou l'huile de tournesol ajoutées à la culture.

9. Procédure selon l'une des revendications précédentes, **caractérisée en ce que** l'acétate est ajoutée à la culture sous forme d'acide acétique ou sous forme de sel de l'acide acétique.

10. Procédure selon revendication 8, **caractérisée en ce que** l'huile végétale est ajoutée au medium dans une concentration entre 0,1 ml/litre et 10 ml/litre.

11. Procédure selon l'une des revendications précédentes, **caractérisée en ce que** l'acétate est ajoutée sous forme d'acétate sodique dans une concentration entre 0,1 g/l et 1 g/litre.

12. Procédure selon l'une des revendications précédentes, **caractérisée en ce que** le contenu de GLA par masse biologique sèche de la culture s'élève à au moins 4 % (f/f), avec une préférence particulière pour la valeur de 5 % (f/f).

13. Procédure selon l'une des revendications précédentes, **caractérisée en ce que** la concentration d'acides linoléiques γ dans la culture s'élève au moins à 300 mg/litre.

14. Procédure selon l'une des revendications précédentes, **caractérisée en ce que** la pureté de l'acide linoléique y par rapport au contenu total des lipides de la culture s'élève au moins à 30 % de la surface totale.

15. Masse biologique sèche prévenant d'une culture de ciliathes, **caractérisée en ce que** la masse biologique sèche contient au moins 4 % (f/f) d'acide linoléique.
